# EUROPEAN PATENT APPLICATION

(11) **EP 3 854 307 A1**
(43) Date of publication of application: **28.07.2021**
(21) Application number: 20152826.2
(22) Date of filing: 21.01.2020
(51) Int. Cl.: A61B 6/10, A61B 6/00, A61B 6/03

(54) **SKIN DOSE MAPPING**

(71) Applicant: Koninklijke Philips N.V., 5656 AG Eindhoven (NL)
(72) Inventor: HOLTHUIZEN, Ronaldus Frederik Johannes, 5656 AE Eindhoven (NL); DOKANIA, Anand Kumar, 5656 AE Eindhoven (NL); JANS, Johannes Cornelis, 5656 AE Eindhoven (NL)
(74) Representative: Philips Intellectual Property & Standards

(57) **Abstract**

The present invention relates to skin dose mapping. In order to provide a facilitated skin dose monitoring, subject situation information data is received that comprises spatial relation data of the subject in relation to an X-ray source and shape of the subject, size of the subject, posture of the subject, position of the subject and/or orientation of the subject. Further, settings of the X-ray source for generating X-ray radiation are received. A subject-model is provided, i.e. generated, based on the subject situation information data. An adapted skin dose map is computed for the subject-model taking into account the spatial relation data and the settings of the X-ray source. The skin dose map is provided as skin dose report.

## Description

### FIELD OF THE INVENTION

The present invention relates to skin dose mapping. The present invention relates in particular to a device for skin dose mapping in medical X-ray radiation, to a medical X-ray radiation system and to a method for skin dose mapping in medical X-ray radiation.

### BACKGROUND OF THE INVENTION

In X-ray imaging, a subject like a patient should not receive more than a certain amount of skin dose, i.e. X-ray radiation during treatment to avoid radiation skin burn. US 9861328 B2 describes X-ray imaging and provides exposure parameter in consideration of dose conditions. So-called load factors (kV, mA, and ms) from the X-ray generator can be used to calculate the cumulative skin dose and skin dose rate of the whole patient. This information may be used to aid a physician in the prevention of skin burns by using alternate projections. Via the skin dose model concept, detailed information about the skin burn risk and warning if the accumulated skin dose of the radiated skin area exceeds the skin burn threshold is provided. However, it has been shown that a required input provided by staff members like a doctor can be cumbersome.

### SUMMARY OF THE INVENTION

There may thus be a need for a facilitated skin dose monitoring.
The object of the present invention is solved by the subject-matter of the independent claims; further embodiments are incorporated in the dependent claims. It should be noted that the following described aspects of the invention apply also for the device for skin dose mapping in medical X-ray radiation, for the medical X-ray radiation system and for the method for skin dose mapping in medical X-ray radiation.

According to the present invention, a device for skin dose mapping in medical X-ray radiation is provided. The device comprises a situation data receiver, a processor and a skin dose reporter. The situation receiver is configured to receive subject situation information data that comprises spatial relation data of the subject in relation to an X-ray source and at least one of the group of shape of the subject, size of the subject, posture of the subject, position of the subject and orientation of the subject. The situation receiver is also configured to receive settings of the X-ray source for generating X-ray radiation. The processor is configured to provide a subject-model based on the subject situation information data. The processor is also configured to compute a skin dose map for the subject-model taking into account the spatial relation data and the settings of the X-ray source. The skin dose reporter is configured to provide the skin dose map as skin dose report.

This provides an improved way of skin dose monitoring that provides accurate information for a current situation. Since the processor provides a subject-model based on the subject situation information data, it is ensured that a correct skin dose prediction for the planned X-ray radiation is available.

According to an example, the subject situation information data is provided as at least one of the group of optical domain image data, ultrasound data and electromagnetic tracking data.

Hence, external sensors can be used for capturing the current situation.

According to an example, the processor is configured to determine if parts of the skin dose map exceed pre-determined threshold dose values. In order to achieve a dose values below the pre-determined threshold dose values, the processor is also configured to compute at least one of the group of: adapted settings of the X-ray source and adapted position of the X-ray source with an adapted spatial relation data of the X-ray source in relation to the subject. The skin dose reporter is configured to provide at least one of the group of the adapted settings of the X-ray source and the adapted position of the X-ray source for the medical X-ray radiation.

According to an example, the processor is configured to generate the subject-model based on the subject situation information data.

Thus, the skin dose monitoring can be provided independent of any data supply from a storage.

According to an example, the processor is provided with a reference model; and the processor is configured to adapt the reference model based on the subject situation information data.

According to the present invention, a medical X-ray radiation system is provided. The system comprises an X-ray source, a device for skin dose mapping in medical X-ray radiation according to one of the preceding examples, and a sensor arrangement with at least one sensor. The at least one sensor is configured to provide the subject situation information data of a subject of interest. The X-ray source is configured to provide the settings. The X-ray source is configured to be operated based on the adapted skin dose map.

According to an example, the system is provided for therapeutic or interventional purposes to provide X-ray radiation for radiation treatment of the subject.

According to an example, the system is a mobile X-ray radiation system.

According to an example, the sensor arrangement comprises at least one optical camera to capture a current spatial relation of a subject and the X-ray source. The camera is a 3D camera.

According to an example, the sensor arrangement comprises at least one ultrasound or infrared sensor to capture the current spatial relation of the subject and the X-ray source.
According to the present invention, also a method for skin dose mapping in medical X-ray radiation is provided. The method comprises the following steps:
- receiving subject situation information data that comprises spatial relation data of the subject in relation to an X-ray source and at least one of the group of shape of the subject, size of the subject, posture of the subject, position of the subject and orientation of the subject;
- receiving settings of the X-ray source for generating X-ray radiation;
- providing a subject-model based on the subject situation information data computing an adapted skin dose map for the subject-model taking into account the spatial relation data and the settings of the X-ray source; and
- providing the skin dose map as skin dose report.

According to an aspect, the exact position and shape of a patient is sensed. Accurate patient distance and shape is used for accurate skin dose mapping. Accurate skin dose mapping will allow accurate dose management to prevent damage to the patient's skin. In an option, graphical visualization is provided to aid in guidance for the user.

The present invention relates to skin dose mapping. In order to provide a facilitated skin dose monitoring, subject situation information data is received that comprises spatial relation data of the subject in relation to an X-ray source and shape of the subject, size of the subject, posture of the subject, position of the subject and/or orientation of the subject. Further, settings of the X-ray source for generating X-ray radiation are received. A subject-model is provided, i.e. generated, based on the subject situation information data. An adapted skin dose map is computed for the subject-model taking into account the spatial relation data and the settings of the X-ray source. The skin dose map is provided as skin dose report.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows an example of a device for skin dose mapping in medical X-ray radiation.
Fig. 2 shows basic steps of a method for skin dose mapping in medical X-ray radiation.
Fig. 3 shows an example of a medical X-ray radiation system.
Fig. 4 shows an example of a sensor of a medical X-ray radiation system.
Fig. 5 shows a schematic setup of an example for X-ray imaging.
Fig. 6 shows a mobile unit as an example for the medical X-ray radiation system in a schematic setup of an example for X-ray imaging.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

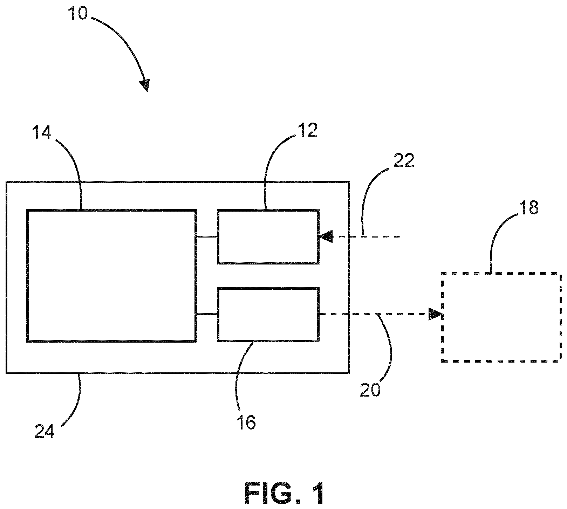

Fig. 1 shows a device 10 for skin dose mapping in medical X-ray radiation. The device 10 comprises a situation data receiver 12, a processor 14 and a skin dose reporter 16. The situation receiver 12 is configured to receive subject situation information data that comprises spatial relation data of the subject in relation to an X-ray source and at least one of the group of shape of the subject, size of the subject, posture of the subject, position of the subject and orientation of the subject; and to receive settings of the X-ray source for generating X-ray radiation. The processor 14 is configured to provide a subject-model based on the subject situation information data and to compute a skin dose map for the subject-model taking into account the spatial relation data and the settings of the X-ray source. The skin dose reporter is configured to provide the skin dose map as skin dose report.

The situation data receiver 12 is also referred to as subject situation data receiver. The situation data receiver 12 is a sort of input or input unit. The processor 14 is a sort of data processor or data processing unit. The skin dose reporter 16 is a sort of output or output unit.

The subject situation information data comprises the information about the subject in relation to an X-ray source to determine which radiation dose is present at the entry of the radiation into the subject, i.e. the radiation on the subject's skin surface.

The subject situation information data comprises data to generate a subject-model. The subject situation information data also comprises data relating to the arrangement of the X-ray source in relation to the subject.

The term "subject" may also be referred to as individual. The subject may further also be referred to as patient, although it is noted that this term does not indicate whether any illness or disease is actually present with the subject.

The term "shape of the subject" relates to the silhouette or contour of the subject. The shape relates to subject's outer surface, i.e. the outer skin surface of the subject.

The term "size of the subject" relates to the actual dimensions of the subject.

The term "posture of the subject" relates to the particular arrangement of the subject, for example when laying on the back or the side, or with angled limbs or arms. The term "position of the subject" relates to the spatial arrangement of the subject, i.e. the location in the room, for example when sitting on a support or standing in front of a detector. The position and posture thus both relate to the particular arrangement of the subject.

The term "orientation of the subject" relates to how the subject is provided in relation to the space or in relation to the X-ray source. The term "spatial relation data of the subject in relation to an X-ray source" relates to the arrangement of the subject and the X-ray source to each other. In particular, the distance of the respective skin portion is provided. "orientation of the subject" and the "spatial relation data of the subject in relation to an X-ray source" thus both relate to the relative arrangement of subject and X-ray source.

The term "skin dose" relates to a dose of X-ray radiation present at the skin's surface of the subject. The skin dose is related to absorbed dose as a dose quantity which is the measure of the energy deposited in matter by ionizing radiation per unit mass. Absorbed dose is used in the calculation of dose uptake in living tissue in both radiation protection (reduction of harmful effects), and radiology (potential beneficial effects for example in cancer treatment).

The term "skin dose map" relates to an indication of the radiation dose across the subject's skin. The skin dose map reflects the shape and size of the subject and the radiation dose for the particular arrangement of subject and X-ray source. The skin dose map is thus a situation-adapted skin dose map.

In an option, an X-ray source situation receiver is provided that is configured to receive X-ray source information data that comprises spatial relation data of an X-ray source in relation to a subject.

The patient situation receiver can also be referred to as patient situation input or patient input. The X-ray source situation receiver can also be referred to as X-ray source situation input or X-ray source input.

In an example, the patient situation receiver and the X-ray source situation receiver are provided as separated receivers or inputs. In another example, the patient situation receiver and the X-ray source situation receiver are provided integrated.

In order to provide the skin dose report, information about the subject and the X-ray source are provided to give exact knowledge of a current situation.

The spatial relation data of the subject in relation to the X-ray source and the shape, size, posture, position and orientation of the subject are provided as current or situation-specific data, thus providing a current or situation-specific skin dose report.

The spatial relation data of the subject in relation to an X-ray source may also be referred to as location data or relative location data.

By providing the subject situation information data and the X-ray source information data, i.e. information about the spatial arrangement, the system is also provided with the patient shape and size, position and, as an option, positions of staff in the examination room.

The term "to provide a subject-model" refers to generating or computing the subj ect-model.

In an example, a 3D optical camera and/or group of sensors are provided to accurately determine the patient shape/size which can be put into the body zone model to determine the exact dose model. Only then a real accurate skin dose estimation can be made as hence the position to the skin will be known.

As a result, support is provided in accurate skin dose mapping. It is also possible to provide competitive positioning with respect to skin dose mapping. The sensors may also provide sufficient spatial information such that additional collision prevention sensors can be removed. In an example, the application is extended to prevent collision of C-arc with the subject or staff members.

The term "to receive settings of the X-ray source for generating X-ray radiation" refers to the provision of the data for the planned operation of the X-ray source, e.g. an X-ray tube.

The settings of the X-ray source for generating X-ray radiation comprise control signals for loading of the X-ray source.

In an example, the subject situation information data relates to a region of interest of the subject that is planned to be subject of X-ray radiation application, such as the thorax, the abdomen area or the lower limbs.

In another example, the subject situation information data relates to the whole subject.

In an option, the subject situation information data is provided as at least one of the group of: i) optical domain image data, ii) ultrasound data and iii) electromagnetic tracking data.

In an option, a combination of at least two of this group above is provided.

In a further option, the subject situation information is provided by any type of sensor that is capable of detecting at least one the criteria of shape of the subject, size of the subject, posture of the subject, position of the subject and orientation of the subject.

Optical domain image data can be provided by cameras, for example, when installed for documentation purposes. The optical domain image data can also be provided by cameras provided for collision control. In an example, video cameras are used providing spatial images of the subject such that the subject's shape and size can be determined. Another example are time-of-flight cameras. In an option, optical markers or other positioning marks such as landmarks are provided on the subject to reconstruct the subject's shape and size from the images of the markers.

Ultrasound data is also suitable, for example, in case of coverage of the subject by cloth drapes.

Electromagnetic tracking data can be provided in relation with markers which also allows to track the subject if covered or hidden by screens or drapes.

In an option, the processor 14 is configured to determine if parts of the skin dose map exceed pre-determined threshold dose values. In order to achieve dose values below the pre-determined threshold dose values, the processor 14 is configured to compute at least one of the group of: adapted settings of the X-ray source, and adapted position of the X-ray source with an adapted spatial relation data of the X-ray source in relation to the subject. The skin dose reporter 16 is configured to provide at least one of the group of the adapted settings of the X-ray source and the adapted position of the X-ray source for the medical X-ray radiation.

In an example, the processor 14 is configured to compute an adapted skin dose map based on at least one of the group of the adapted settings of the X-ray source and the adapted position of the X-ray source with the adapted spatial relation data of the X-ray source in relation to the subject. The adapted skin dose map has dose values below the pre-determined threshold. The skin dose reporter 16 is configured to provide the adapted skin dose map as an adapted skin dose report for the medical X-ray radiation to-be-applied to the subj ect.

In an option, the skin dose reporter 16 comprises a user interface 18 configured to provide at least one of the group of the adapted settings of the X-ray source and the adapted position of the X-ray source for the medical X-ray radiation to the user and to receive a user confirmation for respective adjustment of the settings and position of the X-ray source for the operation of the X-ray source. The interface 18 may be a display. The interface 18 is data-connected, as indicated with dashed line 20.

In an option, the processor 14 is configured to generate the subject-model based on the subject situation information data. In this option, the processor 14 is thus provided to compute the subject-model without the use of a predetermined model and purely based on the subject situation information.

In an option, the processor 14 is provided with a reference model. The processor 14 is configured to adapt the reference model based on the subject situation information data. In this option, the processor 14 is thus provided to fine-tune or adapt the provided model to match the current subject situation. In an example, the reference model is based on a category of the subject chosen from a list of pre-defined categories.

In an option, the reference model is based on pre-taken image data of the subject; for example, based on a previously acquired CT-scan of the patient.

As an option, data input 22 is provided.

In an option, the situation data receiver 12, the processor 14 and the skin dose reporter 16 are provided as separated units.

In another option, the situation data receiver 12, the processor 14 and the skin dose reporter 16 are provided in an integral manner, for example within a common housing 24.

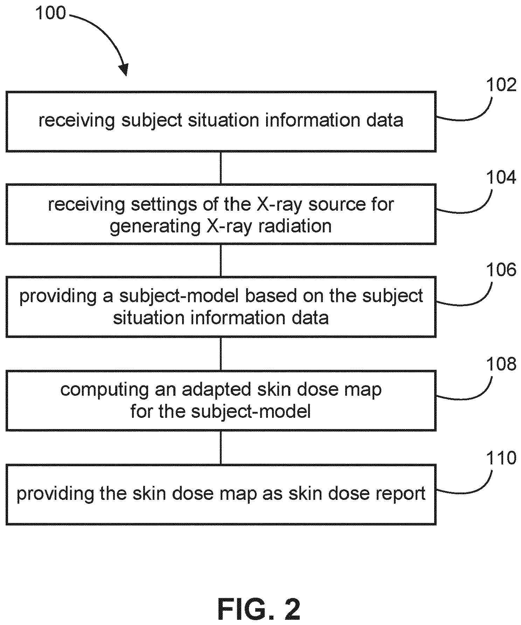

Fig. 2 shows basic steps of a method 100 for skin dose mapping in medical X-ray radiation, the method comprising the following steps:
- In a first step 102, also referred to as step a), subject situation information data is received that comprises spatial relation data of the subject in relation to an X-ray source and at least one of the group of shape of the subject, size of the subject, posture of the subject, position of the subject and orientation of the subject.
- In a second step 104, also referred to as step b), settings of the X-ray source for generating X-ray radiation are received.
- In a third step 106, also referred to as step c), a subject-model based on the subject situation information data is provided.

In a fourth step 108, also referred to as step d), an adapted skin dose map for the subject-model is computed taking into account the spatial relation data and the settings of the X-ray source.
- In a fifth step 110, also referred to as step f), the skin dose map is provided as skin dose report.
In an option, the following steps are provided:
- determining if parts of the skin dose map exceed pre-determined threshold dose values;
- in order to achieve dose values below the pre-determined threshold dose values, computing at least one of the group of: adapted settings of the X-ray source, and adapted position of the X-ray source with an adapted spatial relation data of the X-ray source in relation to the subject; and
- providing at least one of the group of the adapted settings of the X-ray source and the adapted position of the X-ray source for the medical X-ray radiation.
In a further option, also the following steps are provided:
- providing at least one of the group of the adapted settings of the X-ray source and the adapted position of the X-ray source for the medical X-ray radiation to the user, e.g. via a user interface; and
- receiving a user confirmation for respective adjustment of the settings and position of the X-ray source for the operation of the X-ray source.

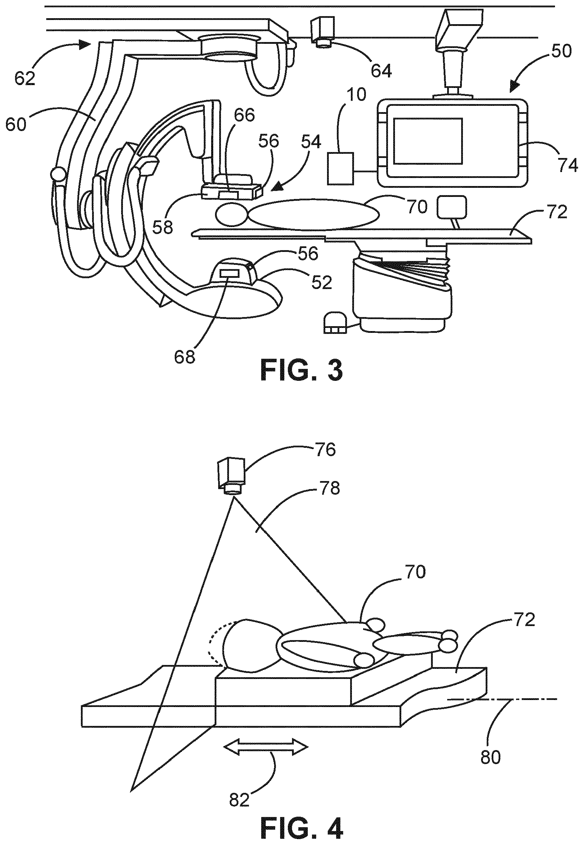

Fig. 3 shows a medical X-ray radiation system 50. The system comprises an X-ray source 52 and an example of the device 10 for skin dose mapping in medical X-ray radiation according to one of the preceding examples. Further, a sensor arrangement 54 with at least one sensor 56 is provided. The at least one sensor 56 is configured to provide the subject situation information data of a subject of interest. The X-ray source is 52 configured to provide the settings. The X-ray source 52 is further configured to be operated based on the adapted skin dose map.

In an example, the X-ray source comprises a controller (not shown) for operating the X-ray radiation, and the controller provides the settings to the situation data receiver.

In a first option, the medical X-ray radiation system is provided for therapeutic or interventional purposes to provide X-ray radiation for radiation treatment of the subject.

In a second option, the medical X-ray radiation system is provided for diagnostic purposes to provide X-ray imaging. For example, an X-ray detector 58 is provided.

In Fig. 3, as an option, a C-arm 60 suspended from a ceiling-rail-system 62 is provided.

In an example, such as for cardiac applications, the X-ray source, also referred to as X-ray tube or tube, is arranged underneath the table. Further, the table position may be known. Still further, the skin of the patient's back may be arranged flat on the table such that the entrance skin dose and actual skin dose in such situation can be calculated accurately. Absorption of tabletop and mattress have to be taken into account for accurate skin dose estimation. Graphical visualization could aid in guidance for the user.

In an option, the system 50 is provided for therapeutic or interventional purposes to provide X-ray radiation for radiation treatment of the subject.

For example, the mobile X-ray system is a C-arm X-ray system, where the X-ray source and an X-ray detector are attached to ends of a movably mounted C-arm structure.

In an example, the X-ray source is mounted to a base that is freely movable across the floor. In an example, the subject support is freely movable across the floor.

In another example, the X-ray source is movably supported by a base that is fixedly mounted.

In an option, the system 50 is a mobile X-ray radiation system.

In an example, the mobile X-ray radiation system is a mobile C-arm system.

In an option, also shown in Fig. 3, the sensor arrangement comprises at least one optical camera 64 to capture a current spatial relation of a subject and the X-ray source. As an option it is provided that the camera 64 is a 3D camera.

It is noted that the 3D camera is provided as an option. Instead of the 3D camera, also other types of cameras are provided, such as 2D cameras.

An example for a camera is a visible light video camera.

Another example for a camera is an infrared video camera.

In an example, the sensor arrangement comprises one or more optical cameras to capture a current spatial relation of a subject and the X-ray source. As an option, a 3D camera is provided. In another option, at least two 2D cameras are provided.

An advantage of 2D/3D optical cameras is that this system that does not require modification of the X-ray system hardware, and therefore is an attractive implementation option. An advantage of this embodiment is that the shape of the patient is determined by the system, therefore increasing the accuracy.

In an option, the sensor arrangement comprises at least one ultrasound 66 or infrared sensor 68 to capture the current spatial relation of the subject and the X-ray source.

In Fig. 3, a subject 70 is arranged on a subject support 72. Further, displays 74 may be arranged in the vicinity of the subject support.

The use of infrared cameras provides the advantage to solve workflow issues as the drapes used in the medical application potentially become semitransparent.

In an option, a combination of sensors, e.g. ultrasound, infrared or optical 3D cameras, is proposed to determine exact positioning and shape of a patient for accurate skin dose mapping. Sensors can be mounted on ceiling and on system to determine shape and position of patient.

As another option, for the tracking technology infrared flash or an alternative tracking system is provided. The system may be equipped with photo-diodes and the position is calculated by measuring the timing difference of laser sweeps originating from boxes attached to the ceiling. Table sensors can be used to detect changes of the patient in the room. Alternatively, ultrasound sensors or radio frequency transmitters are placed on the ceiling and ultrasound or radio frequency sensors are placed on the X-ray system. The position is calculated by measuring the timing difference or signal strength of acoustic/radio frequency pulses originating from boxes attached to the ceiling.

In an example, the mobile X-ray system is tracked in 3D space using a tracker that may be located on the ceiling of the room. The tracking technology may have a large field of view, i.e. capable of detecting of the position variations of the mobile X-ray system in the room, and can be allowed to be less accurate than traditional surgical optical navigation systems. As an option, for the tracking technology, an optical camera system or a structured light optical camera system is provided. For example, one or more 2D/3D optical camera system(s) are located on the ceiling. The position of the X-ray system is calculated based on the 3D shape model of the X-ray system. Table sensors can be used to detect changes of the patient in the room. This system can be used to estimate not only the position of the X-ray system in the room, but also the position and potentially shape of the patient.

In an option, the sensor arrangement is also configured to track staff members and to provide a skin dose report for the staff members.

Fig. 4 shows an example of a sensor of a medical X-ray radiation system. The sensor is provided as a 3D camera 76, e.g. mounted to a ceiling structure. A slit light source may generate a fan-shaped layer 78 or screen of vision. By a relative movement between the 3D camera 76 and the object 70, e.g. along a centerline 80, as indicated by double-arrow 82, the subject can be scanned at least along the region of interest. Thus, spatial relation data of the subject is provided, i.e. spatial information of the subject to provide, e.g. generate, the subj ect-model.

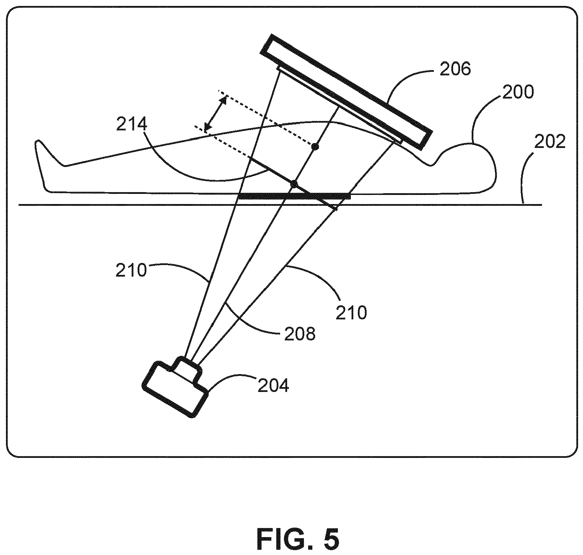

Fig. 5 shows a schematic setup of an example for X-ray imaging. A subject 200 is arranged on a subject support 202. An X-ray source 204 is provided underneath the subject support 202. A detector 206 is arranged on the upper side of the patient. A centerline 208 indicates the central direction of the X-ray beam, being shaped with outer lines 210. A radiation entry plane 212 is indicated below the subject 200. A projection 214 perpendicular to the centerline 208 is indicated. The radiation entry plane 212 is relevant for skin dose control.

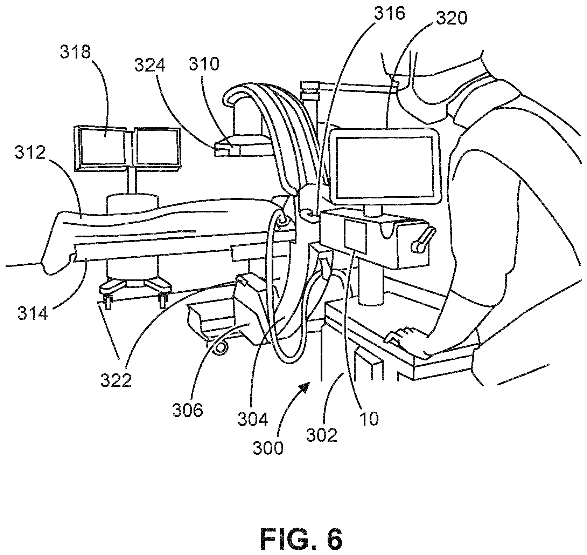

Fig. 6 shows a mobile unit 300 as an example for the medical X-ray radiation system in a schematic setup of an example for X-ray imaging. The mobile unit 300 comprises a base 302, for examples having wheels. Further, a C-arm 304 is attached to the base 302. An X-ray source 306 and an X-ray detector 310 are provided mounted to opposing ends of the C-arm 304. A subject 312 is arranged on a subject support 314. A driving mechanism 316 for moving the C-arm is provided. Further, a device 10 for skin dose mapping in medical X-ray radiation is provided, e.g. integrated into the mobile base unit. Still further, display units 318 are provided in the vicinity to the subject support 314, e.g. mounted to a movable base structure. An additional display / interface 320 may be provided for operating the mobile unit 300. A first sensor 322 is provided attached to a housing of the X-ray source, and a second sensor 324 is provided attached to a housing of the X-ray detector. The two sensors provide the subject situation information data of a subject of interest to the situation data receiver 12 of the device 10 for skin dose mapping in medical X-ray radiation.

In another exemplary embodiment, a computer program is provided enabling a processor to carry out the method of the example above.

In an example, a computer program or program element for controlling an apparatus according to one of the examples above is provided, which program or program element, when being executed by a processing unit, is adapted to perform the method steps of one of the method examples above.

In another exemplary embodiment, a computer readable medium having stored the program element of the above example is provided.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for skin dose mapping in medical X-ray radiation, the device comprising:
- a situation data receiver (12);
- a processor (14); and
- a skin dose reporter (16);
wherein the situation receiver is configured to receive subject situation information data that comprises spatial relation data of the subject in relation to an X-ray source and at least one of the group of shape of the subject, size of the subject, posture of the subject, position of the subject and orientation of the subject; and to receive settings of the X-ray source for generating X-ray radiation;
wherein the processor is configured to provide a subject-model based on the subject situation information data and to compute a skin dose map for the subject-model taking into account the spatial relation data and the settings of the X-ray source; and
wherein the skin dose reporter is configured to provide the skin dose map as skin dose report.

2. Device according to claim 1, wherein the subject situation information data is provided as at least one of the group of:
i) optical domain image data;
ii) ultrasound data; and
iii) electromagnetic tracking data.

3. Device according to one of the preceding claims, wherein the processor is configured to determine if parts of the skin dose map exceed pre-determined threshold dose values; and, in order to achieve dose values below the pre-determined threshold dose values, to compute at least one of the group of:
- adapted settings of the X-ray source; and
- adapted position of the X-ray source with an adapted spatial relation data of the X-ray source in relation to the subject; and
wherein the skin dose reporter is configured to provide at least one of the group of the adapted settings of the X-ray source and the adapted position of the X-ray source for the medical X-ray radiation.

4. Device according to claim 3, wherein the skin dose reporter comprises a user interface configured to provide at least one of the group of the adapted settings of the X-ray source and the adapted position of the X-ray source for the medical X-ray radiation to the user and to receive a user confirmation for respective adjustment of the settings and position of the X-ray source for the operation of the X-ray source.

5. Device according to one of the claims 1 to 4, wherein the processor is configured to generate the subject-model based on the subject situation information data.

6. Device according to one of the claims 1 to 4, wherein the processor is provided with a reference model; and
wherein the processor is configured to adapt the reference model based on the subject situation information data.

7. A medical X-ray radiation system (50), comprising:
- an X-ray source (52);
- a device for skin dose mapping in medical X-ray radiation according to one of the preceding claims; and
- a sensor arrangement (54) with at least one sensor (56);
wherein the at least one sensor is configured to provide the subject situation information data of a subject of interest;
wherein the X-ray source is configured to provide the settings; and
wherein the X-ray source is configured to be operated based on the adapted skin dose map.

8. System according to claim 7, wherein the system is provided for therapeutic or interventional purposes to provide X-ray radiation for radiation treatment of the subject.

9. System according to claim 7 or 8, wherein the system is a mobile X-ray radiation system.

10. System according to claim 7, 8 or 9, wherein the sensor arrangement comprises at least one optical camera (64) to capture a current spatial relation of a subject and the X-ray source;
wherein the camera is a 3D camera.

11. System according to one of the claims 7 to 10, wherein the sensor arrangement comprises at least one ultrasound (66) or infrared sensor (68) to capture the current spatial relation of the subject and the X-ray source.

12. System according to one of the claims 7 to 11, wherein the sensor arrangement is also configured to track staff members and to provide a skin dose report for the staff members.

13. A method (100) for skin dose mapping in medical X-ray radiation, the method comprising the following steps:
- receiving (102) subject situation information data that comprises spatial relation data of the subject in relation to an X-ray source and at least one of the group of shape of the subject, size of the subject, posture of the subject, position of the subject and orientation of the subj ect;
- receiving (104) settings of the X-ray source for generating X-ray radiation;
- providing (106) a subject-model based on the subject situation information data
computing (108) an adapted skin dose map for the subject-model taking into account the spatial relation data and the settings of the X-ray source; and
- providing (110) the skin dose map as skin dose report.

14. A computer program enabling a processor to carry out the method of claim 13.

15. A computer readable medium having stored the program element of claim 14.
